# EUROPEAN PATENT SPECIFICATION

(11) **EP 2 753 295 B1**
(45) Date of publication and mention of the grant of the patent: **29.11.2017**
(21) Application number: 12748694.2
(22) Date of filing: 21.08.2012
(51) Int. Cl.: A61K 8/27, A61K 8/29, A61K 8/39, A61K 8/86, A61Q 17/04, A61K 8/34, A61K 8/36

(54) **A PHOTOPROTECTIVE PERSONAL CARE COMPOSITION**
LICHTSCHUTZKÖRPERPFLEGEZUSAMMENSETZUNG
COMPOSITION DE SOIN PHOTOPROTECTRICE

(30) Priority: 07.09.2011 IN 2491MU2011
(43) Date of publication of application: 16.07.2014
(73) Proprietor: Unilever N.V., 3013 AL Rotterdam (NL); Unilever PLC, London, Greater London EC4Y 0DY (GB)
(72) Inventor: GAURAV, Kumar, Bangalore 560 066 (IN); KUMAR, Praveen, 825301 Jharkhand (IN); PALANISAMY, Bharath, Bangalore 560 066 (IN)
(74) Representative: Corsten, Michael Allan
(86) International application number: PCT/EP2012/066224
(87) International publication number: WO 2013/034427

(56) References cited:
- WO-A1-96/41614
- WO-A1-2008/022946
- WO-A2-03/041677
- GB-A- 2 184 356
- JP-A- 63 275 516

## Description

### Field of the invention

The invention relates to a photoprotective personal care composition. The invention more particularly relates to a sunscreen composition that not only provides high sun protection but does that with minimal or no amount of traditionally used organic sunscreens.

### Background of the invention

Solar radiation includes ultraviolet (UV) radiation, wavelength of which is between 200 nm and 400 nm. Exposure of skin to UV-A (320 to 400 nm) and UV-B (290 to 320 nm) causes various problems like reddening of the skin, localized irritation, sunburn, melanoma and formation of wrinkles. UV radiation is also known to cause damage to hair. Therefore, it is desirable to protect the skin and other keratinous substrates of the human body from the harmful effects of both UV-A and UV-B radiation.

SPF (Sun Protection Factor) is a measure of the protection from solar radiation. In order to achieve this, formulators generally include high amounts of UV-A and UV-B organic sunscreens. The present inventors have found that even with very small amount of organic sunscreen agents or with no organic sunscreens, it is possible to achieve high SPF values with inclusion of a specific class and amount of inorganic sunblocks in combination with specific non-ionic surfactants in a cosmetic base comprising fatty acids.

US5575988A (Knowles, et al. 1996) discloses a combination of sunscreen and insect repellent which is free of organic chemical sunscreens. The composition contains an inorganic micronized substance and DEET (diethyl 3-methyl toluamide) which is applied topically as a lotion or cream.

US2010202985A1 (SenGupta) relates to an emulsion-based sunscreen composition including only inorganic ultraviolet radiation (UV) absorbers known in the art.

Specifically, it relates to sunscreen compositions in the form of oil-in-water (O/W) and water-in-oil (W/O) emulsions that contain inorganic UV-absorbers and an SPF-boosting additive. The said SPF boosting additive is a specific interfacially active polymer.

The above referenced publications do not disclose a personal care photoprotective composition that provides high SPF through use of widely available and inexpensive materials like inorganic sunscreens along with the non-ionic surfactants as claimed herein in combination with a cosmetic base comprising fatty acids and a soap, wherein the cosmetically acceptable base is a vanishing cream base. It is thus an object of the present invention to obviate the drawbacks of the prior art and to provide high SPF photo-protective sunscreen compositions.

Another object of the present invention is to achieve the above object using negligible amounts or no amount of organic sunscreen agents, which are sometimes unstable and with the added advantage that inclusion of low or no organic sunscreens enables low formulation cost.

### Summary of the invention

The first aspect of the present invention provides for a photoprotective personal care composition comprising less than 1% total organic sunscreens by weight of the composition the composition comprising
(i) 1 to 10% inorganic sunscreen having a refractive Index higher than 1.8; and a primary particle size from 5 to 100 nm
(ii) 0.5 to 5% non-ionic surfactant having an HLB value of atleast 13; and,
(iii) a cosmetically acceptable base comprising 6 to 25% fatty acid and 0.1 to 3% soap by weight of the composition, wherein the cosmetically acceptable base is a vanishing cream base.
Another aspect of the invention provides for use of a composition of the invention for providing SPF of at least 10.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. It is more preferably a leave-on product. The composition of the present invention can be in the form of a liquid, lotion, cream, foam, scrub, gel, or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

An advantage of the present invention is that the sunscreen composition is capable of providing an SPF of at least 10, more preferably at least 12, further more preferably at least 15. The invention is capable of providing SPF values as high as 20 and in some cases as high as 25 and in most preferred aspects as high as 30. The composition of the invention includes less than 1% organic sunscreen by weight of the composition. It is preferred that the composition comprises less than 0.8%, preferably less than 0.6%, more preferably less than 0.3%, further more preferably less than 0.1% total organic sunscreens by weight of the composition. In a highly preferred aspect organic sunscreens are absent from the composition of the invention.

An important ingredient that contributes to benefits of the present invention is a non-ionic surfactant. The non-ionic surfactant for use in the composition of the present invention has an HLB value of at least 13, preferably at least 14.5; further more preferably at least 17.5. The HLB value may be as high as 20, preferably as high as 25.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
A value <10 : Lipid soluble (water insoluble)
A value >10 : Water soluble
A value from 4 to 8 indicates an anti-foaming agent
A value from 7 to 11 indicates a W/O (water in oil) emulsifier
A value from 12 to 16 indicates oil in water emulsifier
A value from 11 to 14 indicates a wetting agent
A value from 12 to 15 is typical of detergents
A value of 16 to 20 indicates a solubiliser or hydrotrope

The non-ionic surfactant is preferably selected from the class of alkoxylates e.g. fatty alcohol ethoxylates, alkyl phenol ethoxylates or polyoxyethylene sorbitan alkyl esters. The preferred non-ionic surfactants are ones with at least 9 alkylene oxide groups preferably at least 9 ethylene oxide groups. Preferred non-ionic surfactants are those sold under the brand names of Brij 35 (polyoxyethylene lauryl ether), Brij 58 (polyoxyethylene (20) cetyl ether), Brij700 (polyethylene glycol 4400 octadecyl ether), C12EO9, Tween 21(polyoxyethylenesorbitan monolaurate), Tween20 (polyoxyethylenesorbitan monolaurate), Tween40 (Polyoxyethylenesorbitan monopalmitate), Tween 60 (polyoxyethylene sorbitan monostearate), Triton X165 (octylphenol Ethoxylate), Triton X405 (octylphenol Ethoxylate) or Triton X705 (octylphenol Ethoxylate). The composition of the invention includes 0.5 to 5% non-ionic surfactant by weight of the composition. The non-ionic surfactant is preferably included in 1 to 3% by weight of the composition, more preferably in 1 to 2.5% by weight of the composition.

The composition of the invention comprises 1 to 10% inorganic sunscreen having a refractive Index higher than 1.8. The refractive index of the inorganic sunscreen may be as high as 3.0. Preferred range of refractive index of the inorganic sunscreen is from 1.8 to 2.2. Suitable inorganic sunscreens which may be included as per the above criterion are zinc oxide, titanium dioxide, zinc sulphide, cadmium yellow or Bismuth vanadate. The preferred inorganic sunscreens are titanium dioxide or zinc oxide. The amount of inorganic sunscreen that is incorporated in the composition is preferably 2 to 8%, more preferably 3 to 7% by weight of the composition. The inorganic sunscreens preferably have a primary particle size in the range of 5 to 100 nm. The inorganic sunscreen is preferably hydrophobically coated. Suitable hydrophobic coating materials are aluminium stearate, silicones or ferric stearate.

The composition of the invention comprises a cosmetically acceptable vanishing cream base comprising 6 to 25% fatty acid and 0.1 to 3% soap by weight of the composition. The fatty acids may be saturated or unsaturated fatty acids. C₁₂ to C₂₀ fatty acids are especially preferred in vanishing cream bases, further more preferred being C₁₄ to C₁₈ fatty acids. The fatty acid is preferably a stearic acid or a palmitic acid or a mixture thereof. In creams, the fatty acid is preferably substantially a mixture of stearic acid and palmitic acid. Soaps in the vanishing cream base include alkali metal salt of fatty acids, like sodium or potassium salts. The soap is preferably the potassium salt of the fatty acid mixture. The fatty acid in vanishing cream base is often prepared using hystric acid which is substantially (generally about 90 to 95%) a mixture of stearic acid and palmitic acid. Thus, inclusion of hystric acid and its soap to prepare the vanishing cream base is within the scope of the present invention. It is particularly preferred that the composition comprises at least 6%, preferably at least 10%, more preferably at least 12% fatty acid. The cosmetically acceptable base is usually from 10 to 97%, preferably from 50 to 95% by weight of the composition. The cosmetically acceptable base preferably includes water. Water is preferably included in 35 to 90%, more preferably 50 to 85%, further more preferably 50 to 80% by weight of the composition.

The composition of the invention includes less than 1% total organic sunscreen by weight of the composition. The advantages of the invention are obtained even when no organic sunscreen is present. However the composition may comprise very small amount of organic sunscreen e.g. less than 0.8, preferably less than 0.6, further more preferably less than 0.3, even further more preferably less than 0.1 % organic sunscreen, by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodorant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The invention is now further described by way of the following non-limiting examples.

### Examples

### Example 1 and 2: Effect of inclusion of non-ionic surfactant in compositions comprising no organic sunscreens

Photoprotective personal care vanishing cream compositions (Example -1 and 2) as shown in Table - 1 were prepared and the invitro-SPF was measured using the Optometrics 290S instrument model. The substrate used was an 8 cm Transpore tape procured from 3M Company. The sample was applied at 2 mg/cm². The SPF as measured is shown in Table 1 which is an average of 3 measurements. By three measurements (in this examples and in all examples in this specification) is meant that measurements were carried out on three different samples, each sample being measured at twelve different spots.

**Table 1**

| Ingredients | Example 1 | Example 2 |
|---|---|---|
| Hystric acid | 17.00 | 17.00 |
| KOH | 0.57 | 0.57 |
| Titanium dioxide (MT 100Z) | 5.00 | 5.00 |
| Brij 35(Polyoxyethylene lauryl ether) | 0 | 2.00 |
| Glycerine | 1.00 | 1.00 |
| Cetyl Alcohol | 0.53 | 0.53 |
| Isopropyl myristate | 0.75 | 0.75 |
| Silicone DC - 200/350 | 0.50 | 0.50 |
| Water | To 100 | To 100 |
| SPF | 4.0 | 14.0 |

In Table -1, above, Hystric acid was a mixture of 45% stearic acid and 55% palmitic acid.

Titanium oxide MT100Z was included as particles of 15 nm average particle size coated with aluminium stearate/ aluminium hydroxide which was procured from Tayca.

The data in Table -1 indicates that it is possible by way of the present invention to provide for high SPF when a composition comprises no organic sunscreens but is brought about by synergistic interaction of the inorganic sunscreen and non-ionic surfactant in a cosmetically acceptable base comprising fatty acid.

### Examples 3 to 8: Effect of inclusion of inorganic sunscreens in compositions comprising small amount of organic sunscreens

Various compositions as shown in Table 2 were prepared with small amounts of organic sunscreens (total amounts of less than 1 %) and the effect of inclusion of inorganic sunscreen was studied. The SPF of the various compositions were measured similar to that of Example 1 and are presented in Table - 2 as an average of 3 measurements.

**Table - 2**

| Ingredients | Example 3 | Example 4 | Example 5 | Example 6 | Example 7 | Example 8 |
|---|---|---|---|---|---|---|
| Hystric acid | 17.00 | 17.00 | 17.00 | 17.00 | 17.00 | 17.00 |
| KOH | 0.57 | 0.57 | 0.57 | 0.57 | 0.57 | 0.57 |
| Titanium dioxide (MT 100Z) | 0 | 0 | 0 | 5.00 | 5.00 | 5.00 |
| Brij 35 (Polyoxyethylene lauryl ether) | 2.00 | 2.00 | 2.00 | 2.00 | 2.00 | 2.00 |
| Glycerine | 1.00 | 1.00 | 1.00 | 1.00 | 1.00 | 1.00 |
| Cetyl Alcohol | 0.53 | 0.53 | 0.53 | 0.53 | 0.53 | 0.53 |
| Isopropyl myristate | 0.75 | 0.75 | 0.75 | 0.75 | 0.75 | 0.75 |
| Silicone DC - 200/350 | 0.50 | 0.50 | 0.50 | 0.50 | 0.50 | 0.50 |
| Parsol 1789 (1-[4-(1,1-Dimethylethyl)phenyl]-3-(4-methoxyphenyl) propane-1,3-dione | 0.10 | 0.20 | 0.30 | 0.10 | 0.20 | 0.30 |
| Parsol MCX (2-ethylhexyl-3-(4-methoxyphenyl)-2-propenoate | 0.19 | 0.38 | 0.57 | 0.19 | 0.38 | 0.57 |
| Water | To 100 | To 100 | To 100 | To 100 | To 100 | To 100 |
| SPF | 2.1 | 3.1 | 4.6 | 14.3 | 15.9 | 19.3 |

The data in Table-2 indicates that compositions of the present invention (Examples 6 to 8) provide for high SPF when the compositions comprise the desired amount of inorganic sunscreens and non-ionic surfactant even when small amount of organic sunscreen is present, as compared to similar compositions outside the invention which do not comprise the desired inorganic sunscreen agents (Examples 3 to 5).

### Examples 9 to 23: Compositions comprising non-ionic surfactants within and outside the invention

Various vanishing cream compositions similar to Example 2 were prepared except that various non-ionic surfactants (with different HLB values) were included. The SPF values of these compositions were measured and the data as an average of three measurements is summarized in Table - 3.

**Table 3**

| **Example** | **Surfactant** | **HLB** | **SPF** | **Remarks** |
|---|---|---|---|---|
| 9 | Brij 52 (polyoxyethylene (2) cetyl ether) | 5.3 | 7.0 | Processing issue with the composition |
| 10 | C12EO5 | 9.3 | - | Processing issue with the composition. SPF could not be measured. |
| 11 | Brij S10 (Ethoxy (10) stearyl alcohol) | 12.0 | 7.0 | Processing issue with the composition |
| 12 | C12EO9 | 13.6 | 15.0 | |
| 13 | Brij 58 (polyoxyethylene (20) cetyl ether) | 16.0 | 14.0 | |
| 14 | Brij 35 (Polyoxyethylene lauryl ether) | 16.9 | 14.0 | |
| 15 | Brij 700 (Polyethylene glycol 4400 octadecyl ether) | 18.8 | 14.0 | |
| 16 | Span 20 (Sorbitan monolaurate) | 9.0 | - | Processing issue with the composition. SPF could not be measured. |
| 17 | Tween 21 (Polyoxyethylenesorbitan monolaurate) | 13.1 | 13.0 | |
| 18 | Tween 60 (Polyoxyethylene sorbitan monostearate) | 14.9 | 11.0 | |
| 19 | Tween 40 (Polyoxyethylenesorbitan monopalmitate) | 15.6 | 12.0 | |
| 20 | Tween 20 (Polyoxyethylenesorbitan monolaurate) | 16.9 | 13.0 | |
| 21 | Triton 165 (Octylphenol Ethoxylate) | 15.5 | 12.0 | |
| 22 | Triton 405 (Octylphenol Ethoxylate) | 17.6 | 19.0 | |
| 23 | Trition X 705 (Octylphenol Ethoxylate) | 18.4 | 13.0 | |

The data in Table 3 indicates that compositions as per the invention (Examples 12 to 15 and 17 to 23) provide for high SPF while those outside the invention (Example 9 to 11 and 16) either do not provide the benefit or have some processing issue.

### Examples 24 to 29: Compositions comprising inorganic sunscreens of various particle sizes.

Vanishing cream compositions similar to Example 1 were prepared except that inorganic sunscreens (titanium oxide) of various particle sizes or mixtures of different particles were included, as shown in Table - 4. The SPF values of these compositions were measured and the data is summarized in Table - 4 as an average of 3 measurements.

**Table - 4**

| **Example** | **First Inorganic sunscreen** | **First inorganic sunscreen (wt%)** | **Second Inorganic sunscreen** | **Second inorganic sunscreen (wt%)** | **SPF** |
|---|---|---|---|---|---|
| 24 | MT-100Z (15 nm) | 5 | - | - | 13.0 |
| 25 | MT-500SA (35 nm) | 5 | - | - | 10.0 |
| 26 | MT-700Z (80 nm) | 5 | - | - | 12.0 |
| 27 | MT-100Z (15 nm) | 4 | MT-700Z (80 nm) | 2 | 16.0 |
| 28 | MT-100Z (15 nm) | 3 | MT-700Z (80 nm) | 2 | 16.0 |
| 29 | MT-600B (50nm) | 5 | MT-700Z (80 nm) | 2 | 13.0 |

The data in Table -4 indicates that inorganic sunscreens of various particle sizes preferably in the range of 5 to 100 nm provides the benefit of the invention.

## Claims

1. A photoprotective personal care composition comprising less than 1% total organic sunscreen by weight of the composition the composition comprising
(i) 1 to 10% inorganic sunscreen having a refractive Index higher than 1.8 and a primary particle size from 5 to 100 nm;
(ii) 0.5 to 5% non-ionic surfactant having an HLB value of atleast 13;
(iii) a cosmetically acceptable base comprising 6 to 25% fatty acid and 0.1 to 3% soap by weight of the composition, wherein the cosmetically acceptable base is a vanishing cream base.

2. A composition as claimed in claim 1 wherein said inorganic sunscreen is titanium dioxide or zinc oxide.

3. A composition as claimed in any one of the preceding claims wherein organic sunscreen is less than 0.3%.

4. A composition as claimed in claim 3 wherein the organic sunscreen is less than 0.1%.

5. A composition as claimed in any one of the preceding claims wherein said inorganic sunscreen is present in 2 to 8% by weight of the composition.

6. A composition as claimed in any one of the preceding claims wherein said inorganic sunscreen is hydrophobically coated.

7. A composition as claimed in any one of the preceding claims wherein the non-ionic surfactant is selected from the group consisting of fatty alcohol ethoxylates, alkyl phenol ethoxylates and polyoxyethylene sorbitan alkyl esters.

8. A composition as claimed in claim 7 wherein the non-ionic surfactant has higher than 9 alkylene oxide groups.

9. A composition as claimed in any one of the preceding claims comprising 1 to 3% non-ionic surfactant.

10. A composition as claimed in any one of the preceding claims wherein said fatty acid is stearic acid or palmitic acid or a mixture thereof.

11. A composition as claimed in any one of the preceding claims wherein said soap is a potassium soap.

12. A composition according to any one of claims 1 to 11 for use in providing SPF of at least 10.

## Patentansprüche

1. Körperpflegezusammensetzung mit Lichtschutz, umfassend weniger als 1 Gewichts-% der Zusammensetzung an gesamtem organischen Sonnenschutzmittel, wobei die Zusammensetzung umfasst:
(i) 1 bis 10% anorganisches Sonnenschutzmittel mit einem Brechungsindex von mehr als 1,8 und einer Primärpartikelgröße von 5 bis 100 nm,
(ii) 0,5 bis 5% nicht-ionisches Tensid mit einem HLB-Wert von mindestens 13,
(iii) eine kosmetisch verträgliche Basis, die 6 bis 25% Fettsäure und 0,1 bis 3% Seife, bezogen auf das Gewicht der Zusammensetzung, umfasst, wobei die kosmetisch verträgliche Basis eine verschwindende Creme-Basis darstellt.

2. Zusammensetzung, wie im Anspruch 1 beansprucht, wobei das anorganische Sonnenschutzmittel Titandioxid oder Zinkoxid darstellt.

3. Zusammensetzung, wie in irgendeinem der vorhergehenden Ansprüche beansprucht, wobei das organische Sonnenschutzmittel weniger als 0,3% beträgt.

4. Zusammensetzung, wie im Anspruch 3 beansprucht, wobei das organische Sonnenschutzmittel weniger als 0,1% beträgt.

5. Zusammensetzung, wie in irgendeinem der vorhergehenden Ansprüche beansprucht, wobei das anorganische Sonnenschutzmittel in einer Menge von 2 bis 8 Gewichts-% der Zusammensetzung vorliegt.

6. Zusammensetzung, wie in irgendeinem der vorhergehenden Ansprüche beansprucht, wobei das anorganische Sonnenschutzmittel hydrophob beschichtet ist.

7. Zusammensetzung, wie in irgendeinem der vorhergehenden Ansprüche beansprucht, wobei das nicht-ionische Tensid aus der Gruppe ausgewählt ist, die aus Fettalkoholethoxylaten, Alkylphenolethoxylaten und Polyoxyethylensorbitanalkylestern besteht.

8. Zusammensetzung, wie im Anspruch 7 beansprucht, wobei das nicht-ionische Tensid mehr als 9 Alklenoxidgruppen aufweist.

9. Zusammensetzung, wie in irgendeinem der vorhergehenden Ansprüche beansprucht, die 1 bis 3% nicht-ionisches Tensid umfasst.

10. Zusammensetzung, wie in irgendeinem der vorhergehenden Ansprüche beansprucht, wobei die Fettsäure Stearinsäure oder Palmitinsäure oder eine Mischung davon darstellt.

11. Zusammensetzung, wie in irgendeinem der vorhergehenden Ansprüche beansprucht, wobei die Seife eine Kaliumseife darstellt.

12. Zusammensetzung nach irgendeinem der Ansprüche 1 bis 11 zur Verwendung bei der Bereitstellung eines LSF von mindestens 10.

## Revendications

1. Composition photoprotectrice pour le soin de la personne comprenant moins de 1 % d'écran solaire organique total en masse de la composition, la composition comprenant
(i) de 1 à 10 % d'écran solaire inorganique ayant un indice de réfraction supérieur à 1,8 et une taille de particules primaires de 5 à 100 nm ;
(ii) de 0,5 à 5 % de tensioactif non ionique ayant une valeur HLB d'au moins 13 ;
(iii) une base cosmétiquement acceptable comprenant de 6 à 25 % d'acide gras et de 0,1 à 3 % de savon en masse de la composition, dans laquelle la base cosmétiquement acceptable est une base de crème fondante.

2. Composition selon la revendication 1, dans laquelle ledit écran solaire inorganique est le dioxyde de titane ou l'oxyde de zinc.

3. Composition selon l'une quelconque des revendications précédentes, dans laquelle l'écran solaire organique est inférieur à 0,3 %.

4. Composition selon la revendication 3, dans laquelle l'écran solaire organique est inférieur à 0,1 %.

5. Composition selon l'une quelconque des revendications précédentes, dans laquelle ledit écran solaire inorganique est présent dans de 2 à 8 % en masse de la composition.

6. Composition selon l'une quelconque des revendications précédentes, dans laquelle ledit écran solaire inorganique est hydrophobiquement revêtu.

7. Composition selon l'une quelconque des revendications précédentes, dans laquelle le tensioactif non ionique est choisi dans le groupe constitué d'éthoxylates d'alcools gras, d'éthoxylates d'alkylphénol et d'esters alkyliques de polyoxyéthylène sorbitane.

8. Composition selon la revendication 7, dans laquelle le tensioactif non ionique présente plus de 9 groupes oxyde d'alkylène.

9. Composition selon l'une quelconque des revendications précédentes comprenant de 1 à 3 % de tensioactif non ionique.

10. Composition selon l'une quelconque des revendications précédentes, dans laquelle ledit acide gras est l'acide stéarique ou l'acide palmitique ou un mélange de ceux-ci.

11. Composition selon l'une quelconque des revendications précédentes, dans laquelle ledit savon est un savon de potassium.

12. Composition selon l'une quelconque des revendications 1 à 11 pour une utilisation dans la fourniture d'un SPF d'au moins 10.
